# EUROPEAN PATENT APPLICATION

(11) **EP 1 417 956 A2**
(43) Date of publication of application: **12.05.2004**
(21) Application number: 03250964.8
(22) Date of filing: 18.02.2003
(51) Int. Cl.: A61K 7/48

(54) **Film-forming compositions for topical application**

(30) Priority: 07.11.2002 US 35766; 07.11.2002 US 35765
(71) Applicant: The Procter & Gamble Company, Cincinnati, Ohio 45202 (US)
(72) Inventor: Brooks, Alan (NMN), Woking Surrey GU21 3LN (GB); Russell, Philip Elliott, Egham, Surrey TW20 9RJ (GB); McKay, Barnaby George Robert, Muswell Hill, London N10 1DE (GB)
(74) Representative: Wilding, Richard Alan

(57) **Abstract**

Film-forming compositions for topical application are provided that deliver decreased dry-down times, increased moisturization and improved application and removal characteristics. The film-forming compositions herein comprise a film-forming polymer and a zwitterionic bulking agent. Kits comprising the film-forming compositions of the present invention are also provided herein.

## Description

### TECHNICAL FIELD

The present invention relates to film-forming compositions comprising a film-forming polymer and a zwitterionic bulking agent. The compositions of the present invention provide a film with improved thickness upon application and dry-down. The compositions of the present invention also have reduced dry-down times when topically applied without requiring high ethanol levels, even when high levels of humectants are present.

### BACKGROUND

Film-forming pack compositions provide an excellent vehicle for the delivery of skin actives and moisturization to the skin, especially the face. These compositions are available typically as a pack composition that dries down on the skin to form a film following topical application. These films are also known as facemasks when applied to the face. The film subsequently adheres to the surface of the skin and delivers the actives contained therein over a sustained period, until the facemask is removed by peeling, or washed off.

The facemasks typically incorporate a film-forming polymer such as polyvinyl alcohol (PVA) to create a composition that forms a film after drying down. For example, US 4,743,441 discloses a method of cosmetically treating the skin using a composition comprising a PVA/alkyl vinyl ether copolymer. US 6,368,595 B2 discloses that peelable films comprising a film-forming agent such as PVA are useful for the topical application and delivery of enzymes. These compositions adhere well to the surface of the skin when topically applied, but result in films that are dimensionally thin, stringy and easily punctured following dry down. Also, it is not possible to incorporate high levels of skin moisturizers such as humectants in film-forming compositions, as the humectants bind water in the composition, preventing it from drying and forming a film.

It is possible to increase the thickness and strength of these films by incorporating more film-forming polymer. However, this has application negatives, such as making the composition prior to application very viscous and glue-like, the composition also has increased adhesion to the skin surface following dry-down making it difficult to remove in one piece, and it increases the amount of time required for the composition to dry and form a film to an unacceptable level. It is possible to reduce the drying time by incorporating higher levels of ethanol, but this is not ideal for moisturization of the skin, as the ethanol tends to dry the skin, leaving it feeling tight. Furthermore, these films have little elastic deformation, resulting in the films undergoing plastic deformation, or "stretching", as they are removed, rendering them liable to breakage during removal.

Therefore, it is desirable to provide film-forming compositions for topical application that form dimensionally thick and robust films once dry. It is further desirable to provide film-forming compositions that do not incorporate high levels of film-forming polymer. It is also desirable to provide film-forming compositions for topical application that form dimensionally thick films without increasing the dry-down time required following application. Furthermore, it is desirable to provide film-forming compositions for topical application that have improved moisturization when topically applied to the skin. Similarly, it is desirable to provide film-forming compositions that form a film with a high modulus of elasticity that increases the resilience of the film during removal.

It has surprisingly been found that film-forming compositions comprising a film-forming polymer and a low molecular weight zwitterionic bulking agent act synergistically to provide a composition that forms a relatively thicker and stronger film per unit of polymer following dry down. Furthermore, these compositions have improved drying efficiency without requiring high levels of ethanol. Further still, the compositions of the present invention have improved moisturization and elasticity.

### SUMMARY

Film-forming compositions for topical application comprising a film-forming polymer and a zwitterionic bulking agent having a molecular weight of from greater than 75 to less than 260 are provided. The compositions of the present invention have improved film thickness following dry-down without high levels of film-forming polymer. The compositions also have reduced drying times without high levels of ethanol. Kits comprising the film-forming compositions described herein are also provided.

### DETAILED DESCRIPTION

All weights, measurements and concentrations herein are measured at 25°C on the composition in its entirety, unless otherwise specified.

Unless otherwise indicated, all percentages of compositions referred to herein are weight percentages and all ratios are weight ratios.

Unless otherwise indicated, all molecular weights are weight average molecular weights.

Unless otherwise indicated, the content of all literature sources referred to within this text are incorporated herein in full by reference.

Except where specific examples of actual measured values are presented, numerical values referred to herein should be considered to be qualified by the word "about".

The term "safe and effective amount" as used herein, means an amount of an active ingredient high enough to modify the condition to be treated or to deliver the desired skin care benefit, but low enough to avoid serious side effects, at a reasonable benefit to risk ratio within the scope of sound medical judgment. What is a safe and effective amount of the active ingredient will vary with the specific active, the ability of the active to penetrate through the skin or hair, the age, health condition, and skin or hair condition of the user, and other like factors.

As used herein, "pharmaceutically-acceptable" means that drugs, medications or inert ingredients which the term describes are suitable for use in humans and lower animals without undue toxicity, incompatibility, instability, irritation, allergic response, and the like. As used herein, "cosmetically acceptable" means that ingredients which the term describes are suitable for use in contact with the skin or hair of humans and lower animals without undue toxicity, incompatibility, instability, irritation, allergic response and the like.

As used herein "water-soluble" means materials that are soluble in water at a level of at least 1 gram per litre at 25°C.

As used herein, "glass transition temperature" or "T_{g}" means the temperature at which an amorphous material changes from a brittle vitreous state to a plastic state unless defined otherwise.

The film-forming compositions for topical application of the present invention comprise a water-soluble, film-forming polymer. The film-forming polymer allows for the formation of a peelable mask (peelable film) upon the evaporation of solvent occurring after the topical application of the composition. Furthermore, the film-forming polymer must allow the release and delivery of skin actives to the surface of the skin. Film-forming polymers suitable for use herein may comprise polyvinyl alcohol (PVA), polyvinylpyrrolidone (PVP), polyvinylpyrrolidone (PVP)/VA copolymer, polyvinyl caprolactam, and mixtures thereof. Preferably, the film-forming polymer comprises polyvinyl alcohol (PVA), polyvinyl caprolactam, and mixtures thereof. More preferably, the film-forming polymer comprises polyvinyl alcohol (PVA). Herein, "PVA" includes hydrolysed polyvinyl acetate having a hydrolysis level of at least 75%. Polymers such as carboxyvinyl polymer, acrylic acid polymers, (meth)acrylate polymers and mixtures thereof are not film-forming polymers according to the present invention.

The film-forming compositions herein comprise from 3% to 25% film-forming polymer, preferably 5% to 20%, more preferably 7% to 15% by weight of the composition. Compositions comprising greater than these levels of film-forming polymer are typically too viscous and glue-like before and during application, and generate a film after drying that adheres to the skin too well, making removal difficult. Furthermore, the addition of levels of film-forming polymer above these levels generates a composition that will not readily dry down to form a film without the addition of high levels of ethanol, which results in delipidisation of the skin.

Molecular weights of the polymers are from 10 000 to 2 000 000 preferably 20 000 to 1 000 000, more preferably 30 000 to 500 000. Non-limiting examples of film-forming polymers suitable for use herein include polyvinyl alcohols sold under the trade-name Mowiol (TM) 40-88 (Mᵣ 205,000), 8-88 (MW 67,000) from Clariant, high hydrolysis (greater than 95% hydrolysis) PVAs such as Mowiol 6-98 (Mᵣ 47 000) and 56-98 (Mᵣ 195 000) from Clariant, Celvol 540 (TM) and Celvol 203 (TM) from Celanese. PVP/VA copolymers, Luviskol VA 73 (TM) from BASF, Polyvinyl caprolactam sold as Luviskol Plus (TM), and Kollidone PVP (TM) from BASF. Preferred for use herein are compositions wherein the film-forming polymer comprises greater than 25% PVA having a hydrolysis level of at least 95%. Whilst not being bound by theory, it is believed that the high hydrolysis PVA has a lower affinity for the skin, as it generates are more hydrophilic film, making removal easier.

The film-forming compositions of the present invention also comprise a low molecular weight zwitterionic bulking agent. It has surprisingly been found that the incorporation of a zwitterionic bulking agent and a film-forming polymer as described above provides a film-forming composition for topical application that generates a thick film once dry, without requiring high levels of film-forming polymer. It has also been found that the combination of a zwitterionic bulking agent and film-forming polymer synergistically increase the dimensional thickness of the composition once dry, whilst still providing rapid dry-down when compared with compositions of similar thickness generated using film-forming polymer alone. Furthermore, it has surprisingly been found that the combination of a zwitterionic bulking agent and a film-forming polymer in the film-forming compositions of the present invention increases the elasticity and resilience of the film following dry down.

The interaction of the zwitterionic bulking agent with the film-forming polymer means that low levels of polymer are required to generate a thick and robust film upon drying. Without wishing to be bound by theory, it is believed that the film-forming compositions of the present invention are thickened by the interaction between the film-forming polymer and low molecular weight zwitterionic bulking agent, yet have a more rapid dry-down than those comprising film-forming polymer alone. The improved dry-down is thought to be because the film-forming compositions of the present invention are thickened without increasing the water binding potential of the composition. Generating a film of similar thickness and strength using film-forming polymer alone requires high levels of polymer, subsequently increasing the water-binding potential of the composition. Compositions having these levels of film-forming polymer are glue-like, with very high skin adhesion that are difficult to remove. Furthermore, these compositions also take a very long time to dry down once applied, unless high levels of ethanol are incorporated which is to be avoided with regards to skin moisturization.

Zwitterionic bulking agents suitable for use herein include those with a molecular weight of from greater than 75 to less than 260, preferably from greater than 75 to less than 200 and more preferably from greater than 75 to less than 180.

Zwitterionic bulking agents useful in the present invention comprise quaternary ammonium salts represented by the general formula (I) below: 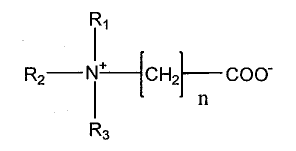 wherein R₁, R₂, and R₃ are independently selected from -H, -CH₃, -CH₂CH₃, and [-CH₂CH(OH)R₄, wherein R₄ is selected from -H, and C1 to C4 alkanes]; and wherein n = an integer from 1 to 3, preferably 1. Preferably R₁, R₂, and R₃ are all -CH₃, and n is 1; or R₁, R₂, and R₃ are all -CH₃, and n is 2; or R₁ and R₂ are -CH₃, R₃ is -H, and n is 1; or R₁ is -CH₃, R₂ and R₃ are -H, and n is 1; or R₁ and R₂ are [-CH₂CH(OH)R₄, R₄ is -H or -CH₃], R₃ is -H, and n is 1.

Further examples of zwitterionic bulking agents and derivatives thereof suitable for use in the present invention include zwitterionic bulking agents conforming to formula (I), wherein any two of R₁, R₂, and R₃ are independently selected from -H, and -CH₃, and the third moiety of R₁, R₂, and R₃ is selected from-(CH₂)mCH₃ wherein m is 4 or 5; and wherein n = an integer from 1 to 3, preferably 1; preferably wherein R₁ and R₂ are -CH₃, R₃ is -(CH₂)mCH₃ wherein m is 4, and n is 1.

Another class of zwitterionic bulking agent derivatives suitable for use in the present invention include cholines conforming to the general formula (II): 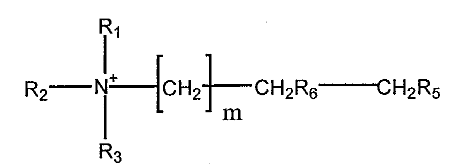 wherein R₁, R₂, R₃ R₄ are defined as in part (i) above; and wherein m is an integer from 0 to 2, and R₅ is selected from PO₄, SO₄ and SO₃, and R₆ is selected from H or OH. Preferred are cholines wherein R₁, R₂, and R₃ are -CH₃; m is 0; R₆ is H and R₅ is PO₄; or taurines wherein R₁, R₂, and R₃ are -CH₃; m is 0; and R₅ is SO₃, R₆ is H, or R₁, R₂, and R₃ are -CH₃; m is 1; R₆ is OH and R₅ is SO₃,

Further zwitterionic bulking agent derivatives suitable for use in the present invention are those comprising proline, carnitine, trimethylamineoxide, tricine, dimethyl proline and mixtures thereof.

Preferred for use herein are zwitterionic bulking agents or derivatives thereof comprising trimethylglycine, dimethyl glycine, sarcosine, trimethyl alanine, tricine, dimethyl proline, bicine, gamma-butyro betaine, trimethylamineoxide, proline, carnitine, and mixtures thereof, more preferably comprising trimethylglycine, tricine and dimethyl glycine; and mixtures thereof.

Non-limiting examples of preferred zwitterionic bulking agents or derivatives for use herein are identified immediately above. Non-limiting examples of preferred zwitterionic bulking agents or derivatives include trimethylglycine hydrate, available as TEGOCARE AP (RTM), from T.H. Goldschmidt (Germany), proline, available as proline, from Huls-Degaussa (Germany), bicine, available as bicine, from Sigma Chemical (USA), and dimethylglycine, available as dimethylglycine from Sigma Chemical (USA).

Preferably, the compositions of the present invention comprise zwitterionic bulking agent or derivative thereof at levels of from 3% to 30% by weight of the composition. More preferably the compositions of the present invention comprise from 5% to 25% of a zwitterionic bulking agent, more preferably still from 7% to 20% by weight of the composition.

Preferred embodiments of the present invention further comprise a glassy material that allow rapid glass formation in the film-forming composition during dry-down. Without wishing to be bound by theory, it is believed that these materials speed up the drying process by generating a glassy hydrate that sequesters remaining water in the film. This allows the formation of a rigid film in the presence of a high level of humectant.

Preferably, the film-forming compositions of the present invention comprise from 0.1% to 15% glassy material, by weight of the composition. More preferably, the compositions of the present invention comprise from 1% to 10%, more preferably from 2% to 8% glassy material. Glassy materials suitable for use herein may comprise low molecular weight, water-soluble materials having a glass transition temperature (T_{g}) of at least 62°C, maltodextrins having a Mᵣ of from 10 000 to 70 000, or mixtures thereof.

Glassy materials suitable for use herein may comprise a low molecular weight, water-soluble material having a T_{g} of at least 62°C. The T_{g} of a material can be determined using standard techniques well known to those skilled in the art, such as Raudonus, J. et al. (2000), "Effect of oligomeric or polymeric additives on glass transition, viscosity and crystallization of amorphous isomalt" Food Research International, 33 pp 41-51. The low molecular weight, water-soluble materials suitable for use herein preferably have a molecular weight (Mᵣ) of from 342 to 1000.

Non-limiting examples of low molecular weight, water-soluble materials suitable for use in the present invention comprise raffinose (Mᵣ: 504, T_{g}: > 100°C), melibiose (Mᵣ 342, T_{g}: 85°C), maltose (Mᵣ 342, T_{g}: 87°C), trehalose (Mᵣ: 342, T_{g}: >100°C), lactose (Mᵣ: 342, T_{g}: 101°C), isomalt (Mᵣ: 344, T_{g}: 62°C), isomaltotriose (Mᵣ: 504, T_{g}: >100°C), melezitose (Mᵣ 504, T_{g}: >100°C), maltotriose (Mᵣ 504, T_{g}: >100°C), stachyose (Mᵣ 666, T_{g}: >100°C), erlose (Mᵣ 504, T_{g}: >100°C), maltotetraose (Mᵣ 666, T_{g}: >100°C) panose (Mᵣ 504, T_{g}: >100°C). Preferred for use herein are sugars and sugar alcohols such as raffinose, melezitose and isomalt.

Also suitable for use herein are glassy materials comprising maltodextrins having a Mᵣ of from 10 000 to 70 000. Below this weight, these materials do not glass the film, whilst above it they produce a film that is too rigid and difficult to remove.

Preferably, the film-forming compositions of the present invention comprise zwitterionic bulking agent and glassy material at a ratio of not greater than 15:1, more preferably not greater than 10:1, and more preferably still not greater than 5:1.

Preferably, the film-forming compositions of the present invention comprise less than 30% ethanol, more preferably less than 20% ethanol, more preferably still from 0.01 % to less than 15% ethanol. It has been found that film-forming compositions comprising more than these levels of ethanol dry the skin by replacing water, leaving the skin feeling tight.

Preferred embodiments of the present invention comprise humectants. Traditionally, it has not been possible to incorporate greater than 2% to 3% humectant in film-forming compositions as compositions comprising greater than this amount did not dry down well enough to form a robust film, and were excessively tacky. This is believed to be due to the water-binding capacity of the humectants slowing water evaporation following application. The film-forming compositions of the present invention can comprise higher levels of humectants that deliver excellent moisturization to the skin, without associated application negatives such as long dry-down times and excessive tackiness. Without wishing to be bound by theory, it is believed that the zwitterionic bulking agent removes water bound to the humectants in the film-forming composition and subsequently releases it more rapidly during dry-down than the humectants themselves, increasing the speed with which the film dries. The use of glass forming materials enhances this effect.

Suitable humectants useful herein include polyhydric alcohols and derivatives, alpha hydroxy acids and derivatives, amides and derivatives, natural extracts, and mixtures thereof.

Preferred humectants, from the viewpoint of boosting moisturisation, are the polyhydric alcohols, amides and mixtures thereof. Suitable polyhydric alcohols for use herein include polyalkylene glycols, including propylene glycol, dipropylene glycol, polypropylene glycol, polyethylene glycol and derivatives thereof, sorbitol, hydroxypropyl sorbitol, phytantriol, erythritol, threitol, pentaerythritol, xylitol, glucitol, mannitol, hexylene glycol, butylene glycol (e.g., 1,3-butylene glycol), butan-1,4-diol, hexane triol (e.g., 1,2,6-hexanetriol), trimethylol propane, neopentyl glycol, glycerine, ethoxylated glycerine and propoxylated glycerine. Preferred polyhydric alcohols of the present invention are polyhydric alcohols with 3 to 9 carbon atoms in the molecule. More preferred polyhydric alcohols include glycerine, butan-1,4-diol, polyethylene glycol, or mixtures thereof. More preferably, the humectant for use in the present invention comprises glycerin.

Suitable amides for use herein comprise sodium 2-pyrrolidone-5-carboxylate (NaPCA), guanidine, lactamide monoethanolamine, acetamide monoethanolamine (AMEA), urea, and mixtures thereof. Preferred for use herein are humectants comprising urea, AMEA, and mixtures thereof. Preferred embodiments of the present invention comprise mixtures of urea and glycerin.

The film-forming compositions of the present invention comprise from 0.1 % to 20% of a humectant, preferably from 2% to 15%, more preferably from 5% to 12% of a humectant.

The film-forming compositions of the present invention may further comprise optional ingredients. Optional ingredients include any material that can be incorporated into the compositions of the present invention in a safe and effective amount without detrimentally affecting the characteristics of the compositions of the present invention. Optional ingredients useful herein include materials selected from the list comprising skin actives, particulate materials, thickening agents, silicones, emollients, surfactants, sunscreening agents, or mixtures thereof.

Some preferred skin active agents for use herein include, but are not limited to, a vitamin B₃ and derivatives, panthenol and derivatives, vitamin E and derivatives, retinoids and derivatives, vitamin C and derivatives, vitamin D and derivatives, caffeine, theobromine, allantoin, alpha-hydroxycarboxylic acids (e.g. glycolic acid, lactic acid, 2-hydroxyoctanoic acid), beta-hydroxycarboxylic acids (e.g. salicylic acid), (alpha- and beta- hydroxycarboxylic acids in combination with salts of glycyrrhic acid, alpha-bisabol, and triclosan), farnesol, glucosamine, hyaluronic acid, aloe vera in any of its variety of forms, pyridoxine, palmityl penta-peptide-3 (available as MATRIXYL (TM) from Sederma Company), pitera (yeast extract), phytosterols (e.g. stigmasterol, sitosterol, brassicasterol, campesterol), flavanoids (e.g. chalcones, chromones, flavones, isoflavones), and mixtures thereof. Preferred skin active agents comprise a Vitamin B₃ compound, a retinoid, a panthenol, a Vitamin E derivative, or mixtures thereof. Incorporation of a skin active in the present compositions enhances the skin benefit properties by providing reduced water loss and/or providing a desquamatory, keratolytic and rejuvenating effect when topically applied.

One class of skin active according to the present invention includes vitamin B₃ compounds. As used herein, "vitamin B₃ compound" includes compounds having the formula: 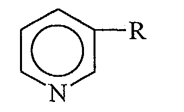 wherein R is - CONH₂ (i.e., niacinamide), - COOH (i.e., nicotinic acid) or -CH₂OH (i.e., nicotinyl alcohol); derivatives thereof; and salts of any of the foregoing. Exemplary derivatives of the foregoing vitamin B₃ compounds include nicotinic acid esters, including non-vasodilating esters of nicotinic acid, nicotinyl amino acids, nicotinyl alcohol esters of carboxylic acids, nicotinic acid N-oxide and niacinamide N-oxide.

Suitable esters of nicotinic acid include nicotinic acid esters of C₁-C₂₂, preferably C₁-C₁₆, more preferably C₁-C₆ alcohols. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (the majority of the general population would not experience a visible flushing response, although such compounds may cause vasodilation not visible to the naked eye). Non-vasodilating esters of nicotinic acid include tocopherol nicotinate and inositol hexanicotinate; tocopherol nicotinate is preferred. When present, the film-forming compositions of the present invention preferably comprise from 0.1 % to 5% of a vitamin B₃ compound.

A further class of skin active according to the present invention comprises panthenol or its derivatives. The panthenol and its derivatives include D-panthenol ([R]-2,4-dihydroxy-N-[3-hydroxypropyl)]-3,3-dimethylbutamide), DL-panthenol, calcium pantothenate, royal jelly, panthetine, pantotheine, panthenyl ethyl ether, pangamic acid, pantoyl lactose and Vitamin B complex. When present, the film-forming compositions of the present invention comprise from 0.1% to 3% panthenol or its derivatives.

A further class of skin active according to the present invention comprises amino acids and their derivatives. Suitable amino acids for use herein include both D- and L- isomers of naturally occurring amino acids. When present, the film-forming compositions of the present invention comprise from 0.1% to 3% amino acids and their derivatives.

The compositions of the present invention may also comprise a retinoid. As used herein, "retinoid" includes all natural and/or synthetic analogs of Vitamin A or retinol-like compounds which possess the biological activity of Vitamin A in the skin as well as the geometric isomers and stereoisomers of these compounds. The retinoid is preferably retinol, retinol esters (e.g., C₂ - C₂₂ alkyl esters of retinol, including retinyl palmitate, retinyl acetate, retinyl propionate), retinal, and/or retinoic acid (including all-trans retinoic acid and/or 13-cis-retinoic acid), more preferably retinoids other than retinoic acid. Such compounds are known and are commercially available from, e.g., Sigma Chemical Company (St. Louis, MO), and Boerhinger Mannheim (Indianapolis, IN). Other retinoids which are useful herein are described in U.S. Patent Nos. 4,677,120, issued Jun. 30, 1987 to Parish et al.; 4,885,311, issued Dec. 5, 1989 to Parish et al.; 5,049,584, issued Sep. 17, 1991 to Purcell et al.; 5,124,356, issued Jun. 23, 1992 to Purcell et al.; and Reissue 34,075, issued Sep. 22, 1992 to Purcell et al. Other suitable retinoids are tocopheryl-retinoate [tocopherol ester of retinoic acid (trans- or cis-), adapalene {6-[3-(1-adamantyl)-4-methoxyphenyl]-2-naphthoic acid}, and tazarotene (ethyl 6-[2-(4,4-dimethylthiochroman-6-yl)-ethynyl]nicotinate). Preferred retinoids are retinol, retinyl palmitate, retinyl acetate, retinyl propionate, retinal and combinations thereof. When present, retinoids will typically be in amounts ranging from 0.005% to 2%, preferably from 0.01 % to 1%.

The compositions of the present invention may further comprise a pH modifiers in an amount that is sufficiently effective to adjust the pH of the composition to fall within a range from 5.5 to 9, preferably from 6.0 to 8.0, more preferably from 6.5 to 7.5. One skilled in the art will understand that the amount of pH modifier to be added will be dependent on the quantity and type of other ingredients selected to make the compositions described herein.

pH modifiers that are suitable for use herein include, but are not limited to: hydroxides; preferred are the sodium salts of these. In addition, the inventive compositions may further comprise buffering agents in an amount that is sufficiently effective to minimize pH drift. Buffering agents that are suitable for use herein include, but are not limited to tris[hydroxymethyl]aminomethane (TRIS), and salts of phosphate, pyrophosphate, citrate, and mellitate, and mixtures thereof, preferably salts of phosphate. It has been found that it is important to provide suitable pH buffering in the film-forming compositions of the present invention to prevent the film-forming polymer from cross-linking and becoming unstable during storage.

The present compositions may comprise at least one silicone phase. The silicone phase can comprise one or more silicone components such as silicone fluids, gums, and mixtures thereof. The total level of silicones in the film-forming compositions of the present invention generally comprises from about 0.01% to about 5%, preferably from about 0.1% to about 2%, more preferably from about 0.1 % to about 1%, of the composition. Without wishing to be bound by theory, it is believed that high levels of silicones hamper the dry-down of the film-forming compositions of the present invention, resulting in the films staying wet and tacky, and never setting properly.

Silicone components can be fluids, including straight chain, branched and cyclic silicones. Suitable silicone fluids useful herein include silicones inclusive of polyalkyl siloxane fluids, polyaryl siloxane fluids, cyclic and linear polyalkylsiloxanes, polyalkoxylated silicones, amino and quaternary ammonium modified silicones, polyalkylaryl siloxanes or a polyether siloxane copolymer and mixtures thereof. The silicone fluids can be volatile or non-volatile. Typically, silicone fluids have a viscosity ranging from about 0.65 to about 600,000 mm².s⁻¹, preferably from about 0.65 to about 10,000 mm².s⁻¹ at 25°C. The viscosity can be measured by means of a glass capillary viscometer as set forth in Dow Corning Corporate Test Method CTM0004, July 29, 1970. Suitable polydimethyl siloxanes that can be used herein include those available, for example, from the General Electric Company as the SF and Viscasil (RTM) series and from Dow Corning as the Dow Corning 200 series. Also useful are essentially non-volatile polyalkylarylsiloxanes, for example, polymethylphenylsiloxanes, having viscosities of about 0.65 to 30,000 mm².s⁻¹ at 25°C. These siloxanes are available, for example, from the General Electric Company as SF 1075 methyl phenyl fluid or from Dow Corning as 556 Cosmetic Grade Fluid. Cyclic polydimethylsiloxanes suitable for use herein are those having a ring structure incorporating from about 3 to about 7 (CH₃)₂SiO moieties. Preferred silicones for use herein comprise 2 mm²/s (2 cst) fluid, available from Dow Corning. These silicone fluids are highly spreadable, resulting in film-forming compositions that have improved application characteristics and, surprisingly, easier removal and improved skin feel. Preferably the film-forming compositions of the present invention comprise from 0.1 % to 1% of a 2 mm²/s (2 cst) silicone fluid.

Another class of silicone components suitable for use in a silicone oil phase herein includes polyether siloxane copolymers. Examples of polyether siloxan copolymers include polydiorganosiloxane-polyoxyalkylene copolymers containing at least one polydiorganosiloxane segment and at least one polyoxyalkylene segment. Suitable polydiorganosiloxane-polyalkylene copolymers are available commercially under the tradenames Belsil (TM) from Wacker-Chemie GmbH, Geschaftsbereich S, Postfach D-8000 Munich 22 and Abil (TM) from Th. Goldschmidt Ltd., Tego House, Victoria Road, Ruislip, Middlesex, HA4 0YL, for example Belsil (TM) 6031 and Abil (TM) B88183. A particularly preferred copolymer fluid blend for use herein includes Dow Coming DC5225C which has the CTFA designation Dimethicone/Dimethicone copolyol.

In preferred embodiments, the silicone fluid is selected from dimethicone, decamethylcyclopentasiloxane, octamethylcyclotetrasiloxane, phenyl methicone, and mixtures thereof.

The silicone components can also comprise silicone gums. Included are non-volatile polyalkyl and polyaryl siloxane gums. In preferred embodiments, a silicone oil phase comprises a silicone gum or a mixture of silicones including the silicone gum. Typically, silicone gums have a viscosity at 25°C in excess of about 1,000,000 mm²s⁻¹. The silicone gums include dimethicones as described in Noll, Walter, Chemistry and Technology of Silicones, New York: Academic Press 1968. Silicone gums are also described in General Electric Silicone Rubber Product Data Sheets SE 30, SE 33, SE 54 and SE 76. Specific examples of silicone gums include polydimethylsiloxane, (polydimethylsiloxane)-(methylvinylsiloxane) copolymer, poly(dimethylsiloxane)(diphenyl)(methylvinylsiloxane) copolymer and mixtures thereof.

Preferably the silicone phase herein comprises a silicone gum incorporated into the composition as part of a silicone gum-fluid blend. Examples of this silicone component is Dow Corning Q2-1503 (85% 5 mm².s⁻¹ Dimethicone Fluid/15% Dimethiconol) and Dow Corning Q2-1501 available from Dow Coming.

Compositions of the present invention may comprise pigments that, where water-insoluble, contribute to and are included in the total level of oil phase ingredients. Pigments suitable for use herein may organic and/or inorganic. Suitable examples include titanium dioxide, and pre-dispersed titanium dioxide, e.g. GWL75CAP (TM) available from Kobo Products (USA).

The film-forming compositions of the present invention may comprise other particulates. Preferably, the compositions of the present invention comprise from about 0.1 % to about 10%, more preferably from 1% to 7%, by weight of particulate materials including pigments. Preferred particulates are free-flowing, materials. Suitable organic particulate materials include those made of polymethylsilsesquioxane, polyamide, polythene, polyacrylonitrile, polyacrylic acid, polymethacrylic acid, microcrystalline cellulose, polystyrene, polytetrafluoroethylene (PTFE), Nylon 12 (e.g. ORGASOL 2002 NAT COS D (TM) from Elf Atochem, and NYLON POLYWL10, available from Optima (England)) and poly(vinylidene chloride). Copolymers derived from monomers of the aforementioned materials may also be used. Inorganic materials include silica and boron nitride and diatomaceous earth. Representative commercially available examples of useful particulate materials herein are TOSPEARL 145 and TOSPEARL 2000 (TMs) available from GE Silicones (New York) which have a median particle size of 4.5 microns and EA-209 (TM), available from Kobo Products (USA) which is an ethylene / acrylic acid copolymer having a median particle size of 10 microns, and aluminium starch octenylsuccinate (available as DRY FLO (TM) from National Starch & Chemical Ltd). Preferred particulates for use herein comprise aluminium starch octenylsuccinate, microcrystalline cellulose, diatomaceous earth or mixtures thereof

The topical compositions of the present invention further comprise a thickening agent. The topical compositions of the present invention comprise from about 0.1% to about 5%, preferably from about 0.1% to about 3%, and more preferably from about 0.25% to about 2%, thickening agent by weight of the composition. Suitable thickening agents include cellulose derivatives, natural gums and derivatives, clays, polyacrylic acid and derivatives, polyacrylamides and derivatives, and mixtures thereof.

Suitable examples of cellulose derivatives include carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, sodium cellulose sulfate, and mixtures thereof, preferably micro-crystalline cellulose. Also useful herein are the alkyl substituted celluloses. In these polymers, the hydroxy groups of the cellulose polymer is hydroyxalkylated (preferably hydroxyethylated or hydroxypropylated) to form a hydroxyalkylated cellulose which is then further modified with a C₁₀-C₃₀ straight chain or branched chain alkyl group through an ether linkage. Typically these polymers are ethers of C₁₀-C₃₀ straight or branched chain alcohols with hydroxyalkylcelluloses. Examples of alkyl groups useful herein include those selected from the group consisting of stearyl, isostearyl, lauryl, myristyl, cetyl, isocetyl, cocoyl (i.e. alkyl groups derived from the alcohols of coconut oil), palmityl, oleyl, linoleyl, linolenyl, ricinoleyl, behenyl, and mixtures thereof. Preferred among the alkyl hydroxyalkyl cellulose ethers is the material given the CTFA designation cetyl hydroxyethylcellulose, which is the ether of cetyl alcohol and hydroxyethylcellulose. This material is sold under the tradename Natrosol (TM) CS Plus from Aqualon Corporation.

Preferred compositions of the present invention comprise a thickening agent comprising polyacrylic acids and derivatives, polyacrylamides and derivatives, carbomers and derivatives and mixtures thereof. Suitable carbomers include carbopol 954. Preferred polyacrylamides are predispersed in a water-immiscible solvent such as mineral oil and the like, containing a surfactant (HLB from about 7 to about 10) which helps to facilitate water dispersibility of the polyacrylamide. Also preferred for use herein is the anionic polymer under the CTFA designation: polyacrylamide and isoparaffin and laureth-7, available under the trade name Sepigel 305 from Seppic Corporation. Also preferred for use herein are the co-polymer compositions commercially available from BASF Corp. under the tradename Luvigel (TM) EM. The film-forming compositions of the present invention preferably comprise from 0.1% to 5% thickening agent.

The compositions of the present invention may be formulated as an oil-in-water emulsion, comprising one or more oil phases in one or more aqueous phases.

The compositions of the present invention may further comprise a surfactant. Suitable surfactants for use herein include cationic, anionic, zwitterionic, amphoteric, non-ionic surfactants, known in the art (see McCutcheon's, Detergents and Emulsifiers, North American Edition (1986), published by Allured Publishing Corporation). Preferred for use herein are non-ionic surfactants. Suitable examples of non-ionic surfactants for use herein include decyl glucoside (the product obtained from the condensation of decyl alcohol with a glucose polymer, available as Plantacare 2000 (TM) from Cognis), polyethylene oxide/polypropylene oxide copolymer, polyethylene oxide/polybutylene oxide copolymer (such as that available from BASF (Germany) under the tradename PLURONIC, particularly, PLURONIC L92), and mixtures thereof. When used, non-ionic surfactants will typically be present in an amount ranging from 0.01% to 2%, preferably from 0.1 % to 1%.

The compositions according to the present invention can optionally include a polymeric cationic conditioning agent. Polymeric cationic conditioning agents are valuable in the compositions according to the present invention for provision of desirable skin feel attributes. If present, the polymeric skin conditioning agent is preferably present at a level from 0.01% to 3%, more preferably from 0.01 % to 2%, more preferably still from 0.01% to 1%. Suitable polymers are high molecular weight materials (mass-average molecular weight determined, for instance, by light scattering, being generally from 2,000 to 5 000 000, preferably from 5 000 to 3 000 000 more preferably from 10 000 to 500 000).

Representative classes of polymers include cationic guar gums, cationic polysaccharides; cationic homopolymers and copolymers derived from acrylic and/or methacrylic acid; cationic cellulose resins, quaternized hydroxy ethyl cellulose ethers; cationic copolymers of dimethyldiallylammonium chloride and acrylamide and/or acrylic acid; cationic homopolymers of dimethyldiallylammonium chloride; copolymers of dimethyl aminoethylmethacrylate and acrylamide, acrylic acid/dimethyldiallylammonium chloride/acrylamide copolymers, quaternised vinyl pyrrolidone acrylate or methacrylate copolymers of amino alcohol, quaternized copolymers of vinyl pyrrolidone and dimethylaminoethylmethacrylamide, vinyl pyrollidone/vinyl imidazolium methochloride copolymers and polyalkylene and ethoxypolyalkylene imines; quaternized silicones, terpolymers of acrylic acid, methacrylamidopropyl trimethyl ammonium chloride and methyl acrylate, and mixtures thereof.

By way of exemplification, cationic polymers suitable for use herein include cationic guar gums such as hydroxypropyl trimethyl ammonium guar gum (d.s. from 0.11 to 0.22) available commercially under the trade names Jaguar C-14-S (TM) and Jaguar C-17 and also Jaguar C-16(TM), which contains hydroxypropyl substituents (d.s. from 0.8-1.1) in addition to the above-specified cationic groups, and quaternized hydroxy ethyl cellulose ethers available commercially under the trade names Ucare Polymer JR-30M, JR-400, LR30M, LR400, Catanal (TM) and Celquat (TM). Other suitable cationic polymers are homopolymers of dimethyldiallylammonium chloride available commercially under the trade name Merquat 100, copolymers of dimethyl aminoethylmethacrylate and acrylamide, copolymers of dimethyldiallylammonium chloride and acrylamide, available commercially under the trade names Merquat 550 and Merquat S, acrylic acid/dimethyldiallylammonium chloride/acrylamide copolymers available under the trade name Merquat 3330, terpolymers of acrylic acid, methacrylamidopropyl trimethyl ammonium chloride and methyl acrylate commercially available under the tradename Merquat 2001, quaternized vinyl pyrrolidone acrylate or methacrylate copolymers of amino alcohol available commercially under the trade name Gafquat, for example Polyquaternium 11, 23 and 28 (quaternized copolymers of vinyl pyrrolidone and dimethyl aminoethylmethacrylate - Gafquat 755N (TM) and quaternized copolymers of vinyl pyrrolidone and dimethyl aminoethylmethacrylamide - HS-100), vinyl pyrrolidone/vinyl imidazolium methochloride copolymers available under the trade names Luviquat FC370 (TM), and polyalkyleneimines such as polyethylenimine and ethoxylated polyethylenimine. Also suitable for use herein are those cationic polymers commercially available as N-HANCE (TM) from Aqualon, and hexadimethrine, Mirapol A15 (TM), Luviquat excellence (TM) and Polycare S (TM). Preferably, the polymeric cationic conditioning agent comprises Mirapol A15 (Polyquaternium 2), hexadimethrine, and mixtures thereof.

Other suitable optional ingredients include perfumes; preservatives, e.g. DMDM hydantoin, benzalkonium chloride, sodium benzoate, ethyl paraben, methyl paraben, propyl paraben, and benzyl alcohol; chelating agents (e.g. ethylenediamine tetraacetic acid (EDTA), and furildioxime), colorants, and mixtures thereof.

The film-forming compositions of the present invention preferably have a viscosity of from 1 Pa.s to 50 Pa.s, more preferably from 2 Pa.s to 25 Pa.s, more preferably still from 3 Pa.s to 10 Pa.s. As used herein, viscosity is measured on the whole composition at 25°C using a Brookfield DVII+ viscometer and spindle CP41 at 1 rpm.

The compositions of the present invention may also be provided in a kit. The kit comprises the film-forming composition of the present invention and at least one applicator. The applicator may comprise a reusable applicator or a single use applicator such as those available as "lollipop" swabs from Qosmedix (New York). Preferably, the applicator comprises a reusable applicator comprising detachable heads. Where the applicator comprises detachable heads, the kit preferably comprises at least one replacement head for the applicator. Furthermore, the kit may comprise instructions indicating use for topical application. Preferably, the kit further comprises means for keeping the hair away from the face. Means for keeping the hair away from the face may comprise any means known to those skilled in the art, non-limiting examples of which include an elastic hair-tie or an arch-shaped head-band made of plastic, rubber or other similar material. The kit may further comprise means for timing the application and dry-down, such as a clock, an hour-glass type timer or other similar device. Timers are useful in kits of the present invention for indicating to the consumer the optimal time for removing the dried face-mask.

The present invention is further directed towards a method of use of the film-forming compositions described herein. The method of use comprises the steps of applying the compositions of the present invention to the skin, preferably the face, at a level of from 20 µl to 300 µl per cm², leaving the composition in contact with the skin surface for a time until it is dry, preferably for at least 15 minutes, more preferably from at least 15 minutes to 30 minutes, more preferably still from 15 minutes to 25 minutes, following which the dried-down film, or "face-pack", is removed from the skin, preferably by peeling off in one piece.

### EXAMPLES

The following are non-limiting examples of embodiments of the present invention. All concentrations are listed as weight percent, unless otherwise specified.

| | **I** | **II** | **III** | **IV** | **V** | **VI** | **VII** |
|---|---|---|---|---|---|---|---|
| Trimethyl glycine | 15 | 20 | 10 | 12 | - | 8 | 12 |
| Bicine | - | - | - | - | 15 | 7 | - |
| Polyvinylalcohol (Mᵣ 31,000) | 3 | - | 2 | 2 | 1 | 3 | 2.5 |
| Polyvinylalcohol (Mᵣ 205,000 mwt) | 10 | 7 | 12 | 8 | 7 | 7 | 8 |
| Polyvinylalcolhol (Mᵣ 30,000) 95% Hydrolysis | - | 3 | - | 4 | 3 | - | - |
| Urea | - | - | - | - | 5 | - | 5 |
| Glycerin | - | - | - | 5 | 3 | 10 | 5 |
| Polyethylene glycol 200 | - | - | - | 5 | 2 | - | - |
| Polyacrylamide & C1213 Isoparaffin & Laureth 7 | - | - | - | 0.5 | 0.7 | 0.4 | - |
| Silicone 2cst | - | - | 0.1 | 0.2 | 0.5 | - | 0.2 |
| Raffinose | - | - | 8 | - | - | - | 3 |
| Isomalt | - | - | - | 4 | - | 3 | - |
| Melezitose | - | - | - | - | 5 | - | - |
| Niacinamide | - | - | 1.0 | 2.0 | - | - | - |
| Panthenol | - | 0.25 | - | 1.0 | - | - | - |
| Decyl glucoside | - | - | 0.15 | 0.2 | - | - | 0.1 |
| Potassium Phosphate | 0.1 | - | 0.2 | 0.1 | 0.2 | - | - |
| Hexadimethrine Chloride | | 0.1 | - | - | - | - | 0.15 |
| Polyquat 2 | | | 0.1 | 0.1 | - | 0.2 | |
| Sodium Benzoate | 0.25 | 0.25 | 0.25 | 0.25 | 0.25 | 0.25 | 0.25 |
| EDTA | 0.1 | 0.1 | 0.1 | 0.1 | 0.1 | 0.1 | 0.1 |
| Ethanol | - | 10 | - | 5 | - | - | 7 |
| pH | 6.5 | 7.5 | 7.0 | 7.5 | 6.0 | 6.0 | 7.0 |
| Water | to 100 | to 100 | to 100 | to 100 | to 100 | to 100 | to 100 |

## Claims

1. A film-forming composition for topical application comprising
a. a film-forming polymer, and
b. zwitterionic bulking agent having a molecular weight of from greater than 75 to less than 260.

2. A film-forming composition for topical application according to claim 1 comprising from 3 to 25% film-forming polymer.

3. A film-forming composition for topical application according to claim 1 or claim 2, wherein the film-forming polymer comprises polyvinyl alcohol, polyvinyl alcohol/vinyl alcohol copolymer, polyvinyl pyrrolidone, polyvinyl pyrrolidone/vinyl acohol copolymer, polyvinyl caprolactam and mixtures thereof, preferably polyvinyl alcohol.

4. A film-forming composition for topical application according to any one of the preceding claims wherein the low molecular weight zwitterionic bulking agent compsrises a quaternary ammonium salt represented by the general formula (I) below: 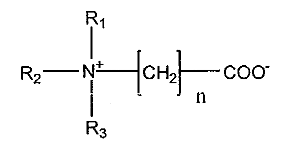 wherein R₁, R₂, and R₃ are independently selected from -H, -CH₃, -CH₂CH₃, and [-CH₂CH(OH)R₄, wherein R₄ is selected from -H, and C1 to C4 alkanes]; and wherein n = an integer from 1 to 3, preferably 1.

5. A film-forming composition for topical application according to any one of the preceding claims comprising from 3% to 30% zwitterionic bulking agent.

6. A film-forming composition according to claim 5 wherein the zwitterionic bulking agent comprises trimethylglycine, proline, bicine, dimethylglycine or mixtures thereof.

7. A film-forming composition for topical application according to any one of the preceding claims further comprising a glassy material.

8. A film-forming composition for topical application according to claim 7 comprising from 0.1% to 15% glassy material.

9. A film-forming composition according to claim 7 wherein the glassy material comprises a low molecular weight, water-soluble material having a glass temperature (T_{g}) of greater than 62°C, maltodextrins having a molecular weight of from 10 000 to 70 000, or mixtures thereof.

10. A film-forming composition for topical application according to claim 9 wherein the glassy material comprises a low molecular weight, water-soluble material having a molecular weight of from 342 to 1000.

11. A film-forming composition for topical application according to claim 10 wherein the glassy material comprises raffinose, melezitose, isomalt, or mixtures thereof.

12. A film-forming composition for topical application according to any one of the preceding claims further comprising from about 0.1% to about 20% humectant.

13. A film forming composition for topical application according to claim 12 wherein the humectant comprises polyhydric alcohol, amides, or mixtures thereof

14. A film-forming composition for topical application according to claim 13 wherein the humectant comprises glycerin.

15. A film-forming composition for topical application according to claim 13 wherein the humectant comprises urea.

16. A film-forming composition for topical application according to claim 13 wherein the humectant comprises a mixture of glycerin and urea.

17. A film-forming composition for topical application according to any one of the preceding claims further comprising a skin active selected from the list comprising vitamin B₃, vitamin B₃ derivatives, panthenol, panthenol derivatives, retinoids, vitamin C, vitamin C derivatives, vitamin D, vitamin D derivatives, vitamin E, vitamin E derivatives, amino acids, or mixtures thereof.

18. A film-forming composition for topical application according to any one of the preceding claims comprising less than 30% ethanol.

19. A film-forming composition for topical application according to any one of the preceding claims comprising from 0.1% to 1% by weight of the composition of a 2 mm²/s silicone fluid.

20. A film-forming composition for topical application according to any one of the preceding claims comprising from 0.1% to 10% by weight of the composition of a particulate material,

21. A film forming composition according to claim 18 wherein the particulate material comprises aluminium starch octenylsuccinate, microcrystalline cellulose, diatomaceous earth or mixtures thereof.

22. A kit comprising a film-forming composition according to claim 1 and at least one applicator.
